# EUROPEAN PATENT APPLICATION

(11) **EP 4 403 131 A1**
(43) Date of publication of application: **24.07.2024**
(21) Application number: 23219704.6
(22) Date of filing: 22.12.2023
(51) Int. Cl.: A61C 7/08, A61K 6/62, A61K 6/887, B33Y 70/00, B33Y 80/00, C08F 222/10, C08L 33/08, B29C 64/124

(54) **PHOTOPOLYMERIZABLE COMPOSITION FOR 3D PRINTER CONTAINING POLYMERIZABLE MONOMER HAVING ALLOPHANATE BOND**

(30) Priority: 27.12.2022 JP 2022210968
(71) Applicant: Shofu Inc., Kyoto-shi, Kyoto 605-0983 (JP)
(72) Inventor: HOSOKAWA, Mamoru, Kyoto, 605-0983 (JP); IMAHORI, Fumio, Kyoto, 605-0983 (JP); FUJIMURA, Hidefumi, Kyoto, 605-0983 (JP)
(74) Representative: Eisenführ Speiser

(57) **Abstract**

[Problem]

To provide a photopolymerizable composition that has excellent formability in 3D printer and can prepare a three-dimensional modeled object with excellent toughness by using optical stereolithography (stereolithography).

[Solution]

To provide a dental photopolymerizable composition for 3D printer, comprising (a) (meth)acrylic polymerizable compound having an allophanate group, (b) (meth)acrylic polymer having one or two (meth)acryloyloxy groups, and (c) photoinitiator.

## Description

### BACKGROUND OF THE INVENTION

### Field of the Invention

The present invention relates to a photopolymerizable composition, in particular to a photopolymerizable composition that has excellent formability in 3D printer and can prepare a three-dimensional modeled object with excellent toughness.

### Description of the Related Art

Many proposals have been made for a method of preparing a three-dimensional modeled object by repeating a step of supplying light energy controlled in required amount to a liquid photo-curable resin and curing it in a thin film form, which is so-called optical three-dimensional modeling method (stereolithography), since the basic practical method was proposed.

The following is typical method for optically preparing a stereolithographic modeled object. The photopolymerizable composition in a container is cured in a predetermined thickness by selectively irradiating a computer controlled light so as to obtain a desired pattern. Then, a photopolymerizable composition is supplied on the upper usurface or the lower surface of the cured layer to the thickness corresponding to one layer and is similarly irradiated with light to be cured. In a commonly used method, these steps are repeated to form a laminated body to prepare a three-dimensional modeled object having a final form.

This method has recently been utilized in various industries because it can easily prepare a desired three-dimensional modeled object in a relatively short time even if the shape of the modeled object is complicated.

In particular, in the field of dental materials, since the shapes of prosthetic devices such as an inlay, a crown and a bridge differ from inical case to clinical case and are complicated, the application of stereolithography has been spread.

In the past, three-dimensional modeled objects prepared by stereolithography was mainly used for preparing prototypes. However, since the precision has been improved with technological development, the application has recently been expanded not only to prototypes but also to the preparation of final products. Therefore, not only modeling accuracy but also excellent strength characteristics has been required.

Typical examples of the prosthesis device prepared by such stereolithography include a dental mouthpiece and a denture base material. There are several types of dental mouthpieces, including one which is called as an orthodontic aligner and is used by wereing on the dentition to correct the alignment of the teeth, one which is called as a dental splint and is used to correct the jaw position, one is used to reduce trauma to the teeth and jawbone during competition in contact sports, and one is used by wearing to treat sleep apnea syndrome and to suppress tooth wear caused by teeth grinding while sleeping. Among these, the use of dental mouthpieces has been rapidly increasing in recent years in the fields of orthodontic aligners and treatment of sleep apnea syndrome.

Denture base material is a material used for the gingival portion of a denture. In recent years, demand for dentures has increased due to the declining birthrate and aging population.

In these dental aligner materials and denture base materials, shape recovery property and toughness are required. If the shape recovery property is impaired, the orthodontic force or shock absorption property are lost, resulting in a material that does not perform its intended function. Further, if the toughness is lost, it becomes easy to be destroyed and needs to be rebuilt frequently.

There are limits in the material viscosity that can be moulded by optical stereolithography (stereolithography). In general, materials with lower viscosity have better formability, but there is a problem in reduced formability because many of the materials that exhibit shape recovery property and toughness have high molecular weight and high viscosity. Therefore, as a whole, it was difficult to prepare a resin composition for use in optical stereolithography which has excellent formability and can prepare a cured product with excellent shape recovery property and toughness.

In this background, as a technology that can prepare a desired cured product with excellent modeling accuracy, for example, a resin composition for optical stereolithography that is compounded with a monofunctional (meth)acrylate monomer and a urethane acrylate in, for exmaple, Japanese Unexamined Patent Application Publication No. 2021-523247. However, in the technology of Japanese Unexamined Patent Application Publication No. 2021-523247, the viscosity of material is high due to the use of urethane acrylate, and optical stereolithography is performed after heating the material to lower its viscosity. Therefore, there is still room for improvement as a material with excellent formability.

In International Publication No. 2010/113600 and Japanese Unexamined Patent Application Publication No. 2017-128688, a composition blended with a monofunctional monomer having an aromatic ring to reduce the viscosity is proposed as a technique for reducing the viscosity of a photocurable resin composition without impairing its curability. However, in International Publication No. 2010/113600 and Japanese Unexamined Patent Application Publication No. 2017-128688, hard materials are assumed and there is still room for improvement in flexibility.

### SUMMARY OF THE INVENTION

### Technical Problem

An object of the present invention is to provide a photopolymerizable composition that has excellent formability in 3D printer and can prepare a three-dimensional modeled object with excellent toughness by using optical stereolithography (stereolithography).

### Solution to Problem

That is, the present invention provides a dental photopolymerizable composition for 3D printer, comprising
(a) (meth)acrylic polymerizable compound having an allophanate group,
(b) (meth)acrylic polymer having one or two (meth)acryloyloxy groups, and
(c) photoinitiator.

### Advantageous Effects of Invention

The photopolymerizable composition of the present invention has excellent formability in 3D printer and can prepare a three-dimensional modeled object with excellent toughness in the case of modeling by optical stereolithography (stereolithography). Therefore, the three-dimensional modeled object of the present invention can be suitably used as a dental material (for example, a dental aligner, a denture base, a mouthpiece).

### DETAILED DESCRIPTION OF THE PREFERRED EMBODIMENTS

The present invention will be described in detail below. The dental photopolymerizable composition for 3D printer of the present invention comprises
(a) (meth)acrylic polymerizable compound having an allophanate group,
(b) (meth)acrylic polymer having one or two (meth)acryloyloxy groups, and
(c) photoinitiator.

In the dental photopolymerizable composition for 3D printer of the present invention, the dental photopolymerizable composition for 3D printer may comprise 30 to 99% by weight of the (a) (meth)acrylic polymerizable compound having an allophanate group, 1 to 70% by weight of the (b) (meth)acrylic polymer having one or two (meth)acryloyloxy groups, and a content of the (c) photoinitiator is 0.01 to 5 parts by weight with respect to 100 parts by weight of the total of the (a) (meth)acrylic polymerizable compound having an allophanate group and the (b) (meth)acrylic polymer having one or two (meth)acryloyloxy groups.

In the dental photopolymerizable composition for 3D printer of the present invention, the (a) (meth)acrylic polymerizable compound having an allophanate group may have a viscosity of 60,000 mPa s or less measured by a rotary rheometer under the conditions of temperature: 23 °C and rotation speed: 20 rpm.

In the dental photopolymerizable composition for 3D printer of the present invention, a glass transition temperature of a cured product of the (a) (meth)acrylic polymerizable compound having an allophanate group may be 120 °C or less.

In the dental photopolymerizable composition for 3D printer of the present invention, the (a) (meth)acrylic polymerizable compound having an allophanate group may have three or more (meth)acryloyloxy groups.

In the dental photopolymerizable composition for 3D printer of the present invention, the (b) (meth)acrylic polymer having one or two (meth)acryloyloxy groups may not contain a urethane structure.

In the dental photopolymerizable composition for 3D printer of the present invention, the (b) (meth)acrylic polymer having one or two (meth)acryloyloxy groups may contain at least one (meth)acrylic polymer having two (meth)acryloyloxy groups.

In the dental photopolymerizable composition for 3D printer of the present invention, the dental photopolymerizable composition for 3D printer may comprise 1 to 25 parts by weight of a (meth)acrylic polymer having one (meth)acryloyloxy group with respect to 100 parts by weight of the total of the (a) (meth)acrylic polymerizable compound having an allophanate group and the (b) (meth)acrylic polymer having one or two (meth)acryloyloxy groups.

In the dental photopolymerizable composition for 3D printer of the present invention, the (b) (meth)acrylic polymer having one or two (meth)acryloyloxy groups may contain a (meth)acrylic polymer containing one or more reactive groups selected from the group consisting of a hydroxyl group and a carboxylic acid group.

In the dental photopolymerizable composition for 3D printer of the present invention, the (c) photoinitiator may be one or more selected from the group consisting of an alkylphenone compound, an acetophenone compound and an acylphosphine oxide compound.

In the dental photopolymerizable composition for 3D printer of the present invention, the dental photopolymerizable composition for 3D printer may have a viscosity of 10,000 mPa s or less measured by a rotary rheometer under the conditions of temperature: 23 °C and rotation speed: 20 rpm.

In the dental photopolymerizable composition for 3D printer of the present invention, a glass transition temperature of a cured product of the dental photopolymerizable composition for 3D printer may be within a range of 30 to 100 °C.

In the dental photopolymerizable composition for 3D printer of the present invention, the dental photopolymerizable composition for 3D printer may further comprise an additive.

In the dental photopolymerizable composition for 3D printer of the present invention, the additive may include one or more selected from the group consisting of a colouring agent, an ultraviolet absorber, a polymerization inhibitor and a fluorescent agent.

In the dental photopolymerizable composition for 3D printer of the present invention, the dental photopolymerizable composition for 3D printer may comprise 0.0001 to 2 parts by weight of the additive with respect to 100 parts by weight of the dental photopolymerisable composition for 3D printers excluding the additive.

The present invention provides a dental product prepared by a stereolithographic 3D printer using a dental photopolymerisable composition for 3D printer of the present invention.

The present invention provides a dental aligner material prepared by a stereolithographic 3D printer using a dental photopolymerisable composition for 3D printer of the present invention.

The present invention provides a denture base material prepared by a stereolithographic 3D printer using a dental photopolymerisable composition for 3D printer of the present invention.

The present invention provides a dental mouthpiece prepared by a stereolithographic 3D printer using a dental photopolymerisable composition for 3D printer of the present invention.

The (a) (meth)acrylic polymerizable compound having an allophanate group has a feature of forming a intramolecular hydrogen bond because of having an allophanate group in its molecule. As a result, it is possible to suppress an intermolecular interaction originating from an urethane bond to lower the the viscosity compared to conventional urethane (meth)acrylate. That is, it is possible to suppress an increase in viscosity and to provide a composition having excellent formability. Furthermore, it has been found that, in the case of using as a material for 3D printer, the (a) (meth)acrylic polymerizable compound having an allophanate group has excellent transparency, and high resilience against deformation from external force to exhibit excellent impact resistance. In other words, it is possible to impart toughness and transparency to a modeled object. In addition, it has been found that by combining the (a) (meth)acrylic polymerizable compound having an allophanate group with the (b) (meth)acrylic polymer having one or two (meth)acryloyloxy groups, it is possible to further suppress an increase in viscosity to provide a composition having excellent formability. In the present specification, the term "allophanate group" refers to the structure "-NH-CO-N-CO₂-". By having an allophanate group in the molecule, it is possible to suppress an increase in viscosity to prepare a composition having excellent formability. In addition, it is possible to impart toughness and transparency to a modeled object.

The (a) (meth)acrylic polymerizable compound having an allophanate group may be contained in an amount of 30 to 99% by weight, 35 to 90% by weight, and 40 to 80% by weight in the photopolymerizable composition. When the amount of the (a) (meth)acrylic polymerizable compound having an allophanate group is less than 30% by weight, there is a case that a problem is caused in that it is difficult to impart toughness to the modeled object. When the amount of the (a) (meth)acrylic polymerizable compound having an allophanate group exceeds 99% by weight, there is a case that a problem is caused in that the viscosity is high and it is difficult to mold.

The (b) (meth)acrylic polymer having one or two (meth)acryloyloxy groups may be contained in an amount of 1 to 70% by weight, 10 to 65% by weight, and 20 to 60% by weight in the photopolymerizable composition. When the amount of the (b) (meth)acrylic polymer having one or two (meth)acryloyloxy groups is less than 1% by weight, there is a case that a problem is caused in that the viscosity is high and it is difficult to mold. When the amount of the (b) (meth)acrylic polymer having one or two (meth)acryloyloxy groups exceeds 70% by weight, there is a case that a problem is caused in that it is difficult to impart toughness.

The (c) photoinitiator may be contained in an amount of 0.01 to 5 parts by weight, 0.5 to 5 parts by weight, and 1.0 to 4 parts by weight with respect to 100 parts by weight of the total of the (a) (meth)acrylic polymerizable compound having an allophanate group and the (b) (meth)acrylic polymer having one or two (meth)acryloyloxy groups. When the amount of the (c) photoinitiator is less than 0.01 parts by weight, there is a possibility that the composition may be difficult to cure and a problem is caused in the formability. When amount of the (c) photoinitiator exceeds 5 parts by weight, there is a possibility that the transparency of the modeled object is poor.

The (a) (meth)acrylic polymerizable compound having an allophanate group preferably has a viscosity of 60,000 mPa s or less measured by a rotary rheometer under the conditions of temperature: 23 °C and rotation speed: 20 rpm, more preferably 50,000 mPa s or less, and most preferably 40,000 mPa s or less. By this, it is possible to suppress an increase in the viscosity of the polymerizable composition and to obtain a photopolymerizable composition with excellent formability.

The (a) (meth)acrylic polymerizable compound having an allophanate group may have a glass transition point of a cured product of 120°C or lower. By appropriately selecting the glass transition point, it is possible to impart optimal toughness to the modeled object.

The (a) (meth)acrylic polymerizable compound having an allophanate group may have three or more (meth)acryloyloxy groups. By this, it is possible to impart appropriate toughness and strength to the modeled object.

The (b) (meth)acrylic polymer having one or two (meth)acryloyloxy groups may be constituted by (meth)acrylate polymerizable monomer not containing urethane structure. In the present specification, "urethane bond" refers to the structure "-NH-CO-O-". By using (meth)acrylate not containing urethane structure, it is possible to suppress the increase in viscosity of the polymerizable composition and impart toughness and transparency to the modeled object.

The (b) (meth)acrylic polymer having one or two (meth)acryloyloxy groups may contain at least one (meth)acrylic polymer having two (meth)acryloyloxy groups. When a (meth)acrylic polymer having two (meth)acryloyloxy groups is not contained, there is a case that a problem is caused in that the modeled object becomes soft.

The (b) (meth)acrylic polymer having one or two (meth)acryloyloxy groups may contain 1 to 25 parts by weight of (meth)acrylic polymer having one (meth)acryloyloxy group. When the amount of the (meth)acrylic polymer having one (meth)acryloyloxy group exceeds 25 parts by weight with respect to the total of 100 parts by weight of the (a) (meth)acrylic polymerizable compound having an allophanate group and the (b) (meth)acrylic polymer having one or two (meth)acryloyloxy groups, there is a possibility that the modeled object becomes soft to cause problem. When the amount of the (meth)acrylic polymer having one (meth)acryloyloxy group is less than 1 part by weight, there is a possibility that it is difficult to improve toughness and to add softness to cause problem.

The (b) (meth)acrylic polymer having one or two (meth)acryloyloxy groups may contain a (meth)acrylic polymer containing one or more reactive groups selected from the group consisting of a hydroxyl group and a carboxylic acid group. By the intermolecular interaction in the polymerizable composition, it is possible to adjust the viscosity and to impart toughness and strength to the modeled object.

The (c) photoinitiator may be one or more selected from the group consisting of an alkylphenone compound, an acetophenone compound and an acylphosphine oxide compound. A plurality of photoinitiators may be combined in consideration of formability and physical properties of the polymerizable composition.

The photopolymerizable composition may have a viscosity of 10,000 mPa s or less measured by a rotary rheometer under the conditions of temperature: 23 °C and rotation speed: 20 rpm. When the viscosity is too high, there is a possibility that a problem is caused in formability.

A glass transition temperature of a cured product of the dental photopolymerizable composition for 3D printer may be within a range of 30 to 100 °C. In the case of this range, it is possible to impart toughness and strength to a modeled object.

The (a) (meth)acrylic polymerizable compound having an allophanate group is not particularly specified, and materials synthesized by known methods can be used. More specifically, it is formed by a reaction between a diisocyanate and an alcohol, and by adding a diisocyanate to a urethane group. The structure may be controlled by changing the hydroxyl equivalent molecular weight of the polyalcohol and the molecular weight of the polyisocyanate.

For example, it is formed by reacting hexamethylene diisocyanate with mono- to hexavalent alcohol having a molecular weight of 32-900 or a mixture of such alcohols. Examples of other isocyanates include tetramethylene diisocyanate, pentamethylene diisocyanate, hexamethylene diisocyanate (HDI), undecamethylene diisocyanate, dodecamethylene diisocyanate, 1-isocyanato-3,3,5-trimethyl-5-isocyanatomethyl cyclohexane (IPDI), 4,4'-bis-(isocyanatocyclohexyl)methane and the like.

Examples of suitable monovalent alcohols include saturated monovalent alcohols such as methanol, ethanol, n-propanol, isopropanol, methoxypropanol and isomer butanol, pentanol, hexanol, octanol, decanol, dodecanol and octadecanol. Examples of polyhydric alcohols include ethylene glycol, propylene glycol, butanediol-1,4, hexanediol-1,6, neopentyl glycol, 2-methylpropanediol-1,3, 2,2,4-trimethylpentane diol-1,3, dimeric aliphatic alcohols, trimeric aliphatic alcohols, glycerol, trimethylolpropane, trimethylolethane, isomeric hexanetriol, pentaerythritol and sorbitol. Unsaturated alcohols such as allyl alcohol, trimethylolpropane diallyl ether, butenediol, and monofunctional alcohols derived from the acids corresponding to unsaturated synthetic and natural fatty acids and acid mixtures of are also suitable.

Specific examples of the (a) (meth)acrylic polymerizable compound having an allophhanate group include a (meth)acrylate resin containing an allophhanate group. The (meth)acrylate resin containing an allophhanate group may include a structure consisting of an allophhanate group represented by the formula "-N(COOR¹)-CO-NH-" in the resin skeleton, and may include a structure represented by the formula "-NH-COOR²-O-CO-C(=CH₂)-R³". The (meth)acrylate resin containing an allophhanate group having these structures may be prepared by reacting a polyisocyanate resin containing allophanate group with an active hydrogen compound having at least one (meth)acrylate group in the molecule.

In the formula "-N(COOR¹)-CO-NH-", R¹ represents an aliphatic group, an alicyclic group, or an aromatic hydrocarbon group in which the total carbon number is 1 to 40. However, these hydrocarbon groups may have a branch or a substituent.

Further, in the formula "-NH-COOR²-O-CO-C(=CH₂)-R³", R² represents an organic group having the total carbon number of 1 to 50, and R³ represents hydrogen atom or methyl group. However, the organic group R² may have a branch or a side chain.

### <Polyisocyanate resin containing allophanate group>

The polyisocyanate resin containing allophanate group may be prepared by reacting a monoalcohol with an organic polyisocyanate prepolymer under specific condition.

### <<Preparation of organic polyisocyanate prepolymer >>

The organic polyisocyanate prepolymer means a prepolymer having a isocyanate group and may usually be prepared by reacting an isocyanate compound with a hydroxy group-containing compound.

The isocyanate compound used in the preparation reaction of the organic polyisocyanate prepolymer may usually be classified into a diisocyanate compound and a polyisocyanate compound. The polyisocyanate compound means an isocyanate compound having three or more isocyanate groups.

Specific examples of the diisocyanate compound include
an aliphatic diisocyanate compound such as trimethylene diisocyanate, tetramethylene diisocyanate, hexamethylene diisocyanate (HDI), pentamethylene diisocyanate, 1,2-propylene diisocyanate, 1,2-butylene diisocyanate, 2,3-butylene diisocyanate, 1,3-butylene diisocyanate, 2,4,4- or 2,2,4-trimethyl hexamethylene diisocyanate and 2,6-diisocyanate methylcaproate;
an alicyclic diisocyanate compound such as 1,3-cyclopentene diisocyanate, 1,4-cyclohexane diisocyanate, 1,3-cyclohexane diisocyanate, 3-isocyanatomethyl-3,5,5-trimethylcyclohexyl isocyanate, 4,4'-methylene bis (cyclohexylisocyanate), methyl-2,4-cyclohexane diisocyanate, methyl-2,6-cyclohexane diisocyanate, 1,3-bis (isocyanatomethyl) cyclohexane, 1,4-bis (isocyanatomethyl) cyclohexane, 1,3-diisocyanatomethyl cyclohexane (H6 XDI) and isophorone diisocyanate (IPDI);
an aromatic aliphatic diisocyanate compound such as 1,3- or 1,4-xylylene diisocyanate, mixtures of the two compounds, ω,ω'-diisocyanato-1,4-diethylbenzene, 1,3- or 1,4-bis (1-isocyanato-1-methylethyl) benzene and mixtures of the two compounds; and
an aromatic diisocyanate compound such as m-phenylene diisocyanate, p-phenylene diisocyanate, 4,4'-diphenyl diisocyanate, 1,5-naphthalene diisocyanate, 4,4'-diphenylmethane diisocyanate, 2,4-tolylene diisocyanate, 2,6-tolylene diisocyanate, 4,4'-toluidine diisocyanate, 4,4'-diphenylether diisocyanate and toluene diisocyanate (TDI).

Specific examples of the polyisocyanate compound include
an aliphatic polyisocyanate compound such as lysine ester triisocyanate, 1,4,8-triisocyanato octane, 1,6,11-triisocyanato undecane, 1,8-diisocyanato-4-isocyanato methyloctane, 1,3,6-triisocyanato hexane, 2,5,7-trimethyl-1,8-diisocyanato-5-isocyanato methyloctane;
an alicyclic polyisocyanate compound such as 1,3,5-triisocyanato cyclohexane, 1,3,5-trimethylisocyanato cyclohexane, 2-(3-isocyanato propyl)-2,5-di(isocyanato methyl)-bicyclo(2.2.1)heptane, 2-(3-isocyanato propyl)-2,6-di(isocyanato methyl)-bicyclo(2.2.1)heptane, 3-(3-isocyanato propyl)-2,5-di(isocyanato methyl)-bicyclo(2.2.1)heptane, 5-(2-isocyanato ethyl)-2-isocyanato methyl-3-(3-isocyanato propyl)-bicyclo(2.2.1)heptane, 6-(2-isocyanato ethyl)-2-isocyanato methyl-3-(3-isocyanato propyl)-bicyclo(2.2.1)heptane, 5-(2-isocyanato ethyl)-2-isocyanato methyl-2-(3-isocyanato propyl)-bicyclo(2.2.1)heptane and 6-(2-isocyanato ethyl)-2-isocyanato methyl-2-(3-isocyanato propyl)-bicyclo(2.2.1)heptane;
an aroaliphatic polyisocyanate compound such as 1,3,5-triisocyanato methylbenzene; and
an aromatic polyisocyanate compound such as triphenylmethane-4,4',4"-triisocyanate, 1,3,5-triisocyanato benzene, 2,4,6-triisocyanato toluene and 4,4'-diphenylmethane-2,2',5,5'-tetraisocyanate.

These isocyanate compounds may be used alone or in combination of two or more. Among these isocyanate compounds, hexamethylene diisocyanate (HDI), 1,3-diisocyanato methylcyclohexane (H6XDI), and isophorone diisocyanate (IPDI) are preferable.

Specific examples of the above described hydroxyl group-containing compound used in the preparation reaction of the organic polyisocyanate prepolymer include
monoalcohols (monohydric alcohols) such as methanol, ethanol, propanol, butanol, an alkanol having 5 to 38 carbon atoms, an alkenyl alcohol having 3 to 36 carbon atoms (for example, 2-propen-1-ol), an alkadienol having 6 to 8 carbon atoms (for example, 3,7-dimethyl-1,6-octadien-3-ol) and an aliphatic unsaturated alcohol having 9 to 24 carbon atoms other than the above described;
dialcohols (dihydric alcohols) such as ethylene glycol, propanediol, 1,4-butanediol, 1,3-butanediol, 1,2-butanediol, 1,6-hexanediol, methylpentanediol, neopentyl glycol, 3,3-dimethylolheptane, alkanediol having 7 to 22 carbon atoms, diethylene glycol, triethylene glycol, dipropylene glycol, cyclohexane dimethanol, alkane-1,2-diol having 17 to 20 carbon atoms, hydrogenated bisphenol A, 1,4-dihydroxy-2-butene, 2,6-dimethyl-1-octene-3,8-diol and bisphenol A;
trialcohols (trihydric alcohols) such as glycerin, 2-methyl-2-hydroxymethyl-1,3 -propanediol, 2,4-dihydroxy-3 -hydroxymethylpentane, 1,2,6-hexanetriol, 1,1,1-tris (hydroxymethyl) propane, 2,2-bis (hydroxymethyl)-3-butanol, and other aliphatic triols having 8 to 24 carbon atoms;
tetrahydric or higher alcohols such as tetramethylolmethane, D-sorbitol, xylitol, D-mannitol and D-mannite;
polyester polyols such as neopentyl pivalate glycol ester;
polyether polyols such as polyethylene glycol, polypropylene glycol and polytetramethylene ether glycol; and
polycarbonate polyols such as polyhexamethylene carbonate glycol.

Among these compounds having a hydroxyl group, monoalcohols and dialcohols are preferable. Moreover, these compounds having a hydroxyl group may be used alone or in combination of two or more.

The organic polyisocyanate prepolymer can be prepared by reacting the above described monoalcohol with the above described isocyanate compound in an amount that gives an excess isocyanate group equivalent with respect to twice equivalent of the hydroxyl group equivalent of the monoalcohol in the case that the hydroxyl group compound is a monoalcohol, and can be prepared by reacting the above described hydroxyl group-containing compound with the above described isocyanate compound in an amount that gives an excess isocyanate group equivalent with respect to the hydroxyl group equivalent of the hydroxyl group-containing compound in the case that the hydroxyl group compound is other than a monoalcohol, and if necessary, by removing unreacted isocyanate compound.

### <<Preparation of Polyisocyanate resin containing allophanate group>>

The polyisocyanate resin containing allophanate group can be prepared by reacting an organic polyisocyanate prepolymer with a monoalcohol under specific reaction conditions, and as the method for preparing the polyisocyanate resin containing allophanate group,it is possible to use the methods described, for example, in Japanese Unexamined Patent Application Publication No. H5-209038 and Japanese Unexamined Patent Application Publication No. H8-188566.

That is, the polyisocyanate resin containing allophanate group can prepared by an allophanatization reaction of a monoalcohol with an organic polyisocyanate prepolymer in an amount that gives an excess isocyanate group equivalents with respect to the hydroxyl group equivalents in the monoalcohol in the presence of a catalyst and removing unreacted organic polyisocyanate prepolymer.

By these reactions, a monoalcohol can be bonded to the organic polyisocyanate prepolymer via the allophanate group to improve the solubility in acrylic resins and polyester resins dramatically. In addition, by the skeleton derived from monoalcohol, it is possible to impart flexibility to the cured product prepared by using the resin to achieve both flexibility and scratch resistance.

The monoalcohol used in the above preparing process may have a polar group such as an unsaturated bond, an ether group and an ester group. Moreover, the above-mentioned monoalcohol can be appropriately selected and used depending on other resins to be made compatible.

Among these monoalcohols, monoalcohols having 1 to 40 carbon atoms are preferable. By using a monoalcohol having a carbon number within the above range, it is possible to achieve both flexibility and scratch resistance of the cured coating film.

Examples of these monoalcohols include
aliphatic, alicyclic and aromatic aliphatic alcohols shuch as methanol, ethanol, n-propanol, isopropanol, butanol isomers, allyl alcohol, pentanol, hexanol, heptanol, 2-ethylhexanol, n-octanol, nonanol, n-decanol, cyclopentanol, cyclohexanol, furfuryl alcohol, benzyl alcohol;
an ether group-containing monoalcohol that is an addition polymer of the above described monoalcohol and an alkylene oxide such as ethylene oxide or propylene oxide (a random and/or block copolymer of two or more alkylene oxides);
an ester group-containing monoalcohol that is addition polymers of low molecular weight monoalcohol and lactones such as ε-caprolactone and δ-valerolactone; and
an ester group-containing monoalcohol that is an adduct of a monocarboxylic acid such as acetic acid, propionic acid, or benzoic acid and an alkylene oxide.

Further, in addition to the above described monoalcohol having 1 to 40 carbon atoms, a small amount of polyfunctional alcohol such as dialcohol or trialalcohol may be used in combination. Furthermore, in addition to these, an active hydrogen compound such as thiols, oximes, lactams, phenols, and β-diketones may also be used in combination as necessary.

Furthermore, before the allophanate reaction between the organic polyisocyanate prepolymer and the monoalcohol, a urethane group may be generated by prereaction of some or all of the hydroxyl groups present in the monoalcohol with some of the isocyanate groups present in the organic polyisocyanate compound.

The using ratio of the organic polyisocyanate prepolymer and the monoalcohol is usually in the range of 5 to 100, preferably in the range of 10 to 50 in terms of the molar ratio of isocyanate groups present in the organic polyisocyanate prepolymer to hydroxyl groups present in the monoalcohol ([isocyanate groups in the prepolymer]/[hydroxyl groups in the monoalcohol]). When the molar ratio of isocyanate groups to hydroxyl groups is within the above range, the content of allophanate groups is appropriate, and a resin having high solubility with other resins can be obtained.

In the reaction between the monoalcohol and the organic polyisocyanate compound, a solvent may be used or may not be used. In the case of using a solvent, it is necessary to use a solvent that does not have reactive activity with respect to an isocyanate group.

As the catalyst used for the reaction between the monoalcohol and the organic polyisocyanate prepolymer, a catalyst may be used that allows easy control of the reaction, causes less coloring of the final product, and produces less dimer with poor thermal stability.

Specific examples of these catalysts include
tetraalkylammonium hydroxide such as tetramethylammonium, tetraethylammonium, tetrabutylammonium, trimethylbenzylammonium and organic weak acid salts thereof;
trialkyl hydroxyalkylammonium hydroxide such as trimethyl hydroxypropylammonium, trimethyl hydroxyethylammonium, triethyl hydroxypropylammonium, triethyl hydroxyethylammonium and organic weak acid salts thereof;
alkyl carboxylate of alkyl carboxylic acid such as acetic acid, caproic acid, octylic acid and myristic acid, and alkali metal;
alkyl carboxylic acid metal salt of the above alkyl carboxylic acid and a metal such as tin, zinc, or lead;
a chelate compound of β-diketone and metals such as aluminum acetylacetone and lithium acetylacetone;
Friedel-Crafts catalyst such as aluminum chloride and boron trifluoride;
various organometallic compounds such as titanium tetrabutyrate, tributylantimony oxide; and
an aminosilyl group-containing compound such as hexamethylsilazane.

Among these catalysts, a quaternary ammonium compound such as tetraalkylammonium hydroxide and their organic weak acid salt, and trialkylhydroxyalkylammonium hydroxide and their organic weak acid salt are preferably used.

Catalysts are usually used in the range of 0.0001 to 1% by weight with respect to the organic polyisocyanate, although depending on the type and reaction temperature. Allophanation reactions are usually carried out in the temperature range of 20 to 160 °C, preferably 40 to 100 °C.

The desired polyisocyanate resin can be prepared by adding an acidic substance such as phosphoric acid, benzoyl chloride, monochloroacetic acid, or dodecylbenzenesulfonic acid to the reaction mixture as a catalyst inactivator to deactivate the catalyst when the amount of residual NCO reaches the desired amount, and then removing unreacted organic polyisocyanate prepolymer by means of thin film distillation, for example.

The polyisocyanate resin having an allophanate group obtained in this way may be made into a resin that can be mixed with other resins in any ratio by appropriately selecting the monoalcohol that is the raw material, and it is possible to provide a resin that does not become cloudy below room temperature.

The above described polyisocyanate resin containing allophanate group may also be prepared by reacting the isocyanate compound used in the preparation of the above described organic polyisocyanate prepolymer with a monoalcohol, which is a compound having a hydroxyl group, under conditions where the isocyanate compound is in large excess.

### <Preparation of (meth)acrylate resin containing allophanate group>

The (meth)acrylate resin containing an allophhanate group may be prepared by reacting the above described polyisocyanate resin containing allophanate group with an active hydrogen compound having at least one (meth)acrylate group in the molecule. As the active hydrogen compound having at least one (meth)acrylate group used in this reaction, a compound containing a (meth)acrylate group and a hydroxyl group may be suitably used.

The (a) (meth)acrylic polymerizable compound having an allophanate group is not particularly specified, and materials synthesized by known methods can be used. Specific examples include hexane, 1,6-diisocyanate, homopolymer, 2-hydroxyethyl acrylate block, hexane, 1,6-diisocyanate, homopolymer, 2-hydroxyethyl acrylate, propylene glycol monoacrylate block, and the like. These may be used alone or in combination of two or more.

Specific examples of the (a) (meth)acrylic polymerizable compound having an allophanate group represented by the general formula "R¹N(COOR²)CONHCOR³" include
a compound in which R¹ is a trifluoromethylbenzene group, R² is a methylbenzene group and R³ is a morpholine group,
a compound in which R¹ is a trifluoromethylbenzene group, R² is a methylbenzene group and R³ is a methoxy group,
a compound in which R¹ is a trifluoromethylbenzene group, R² is a methylbenzene group and R³ is a hexanol group,
a compound in which R¹ is a trifluoromethylbenzene group, R² is a benzene group and R³ is a morpholine group,
a compound in which R¹ is a trifluoromethylbenzene group, R² is a chlorobenzene group and R³ is a decanol group, and
a compound in which R¹ is a dichlorobenzene group, R² is a benzene group and R³ is a toluidine group.

Specific examples of the (a) (meth)acrylic polymerizable compound having an allophanate group having three or more (meth)acryloyloxy groups include the compounds used in the Examples of the present spesification.

As the (b) (meth)acrylic polymer having one or two (meth)acryloyloxy groups, specific examples of the (meth)acrylic polymer having two (meth)acryloyloxy groups include ethylene glycol dimethacrylate, diethylene glycol dimethacrylate, triethylene glycol dimethacrylate, polyethylene glycol dimethacrylate, 2,2-bis [4-(methacryloxyethoxy) phenyl] propane, tricyclodecane dimethanol dimethacrylate, 1,10-decanediol dimethacrylate, 1,6-hexanediol dimethacrylate, 1,9-nonanediol dimethacrylate, neopentyl glycol dimethacrylate and 2-hydroxy-1,3-dimethacryloxypropane. Among these, triethylene glycol dimethacrylate and 2,2-bis (4-methacryloxy polyethoxyphenyl) propane are preferable.

As the (b) (meth)acrylic polymer having one or two (meth)acryloyloxy groups, specific examples of (meth)acrylic polymer having one (meth)acryloyloxy group include o-benzyl (meth)acrylate, m-phenoxybenzyl (meth)acrylate, p-phenoxybenzyl (meth)acrylate, 2-(o-phenoxyphenyl) ethyl (meth)acrylate, 2-(m-phenoxyphenyl) ethyl (meth)acrylate, 2-(p-phenoxyphenyl) ethyl (meth)acrylate, 3-(o-phenoxyphenyl) propyl (meth)acrylate, 3-(m-phenoxyphenyl) propyl (meth)acrylate,
3-(p-phenoxyphenyl) propyl (meth)acrylate, 4-(o-phenokyphenyl) butyl (meth)acrylate, 4-(m-phenokyphenyl) butyl (meth)acrylate, 4-(p-phenokyphenyl) butyl (meth)acrylate, 5-(o-phenokyphenyl) pentyl (meth)acrylate, 5-(m-phenokyphenyl) pentyl (meth)acrylate, 5-(p-phenokyphenyl) pentyl (meth)acrylate, 6-(o-phenokyphenyl) hexyl (meth)acrylate, 6-(m-phenokyphenyl) hexyl (meth)acrylate, 6-(p-phenokyphenyl) hexyl (meth)acrylate, ethyl methacrylate, n-butyl methacrylate, isobutyl methacrylate, tert-butyl methacrylate, 2-ethylhexyl methacrylate, isodecyl methacrylate, alkyl methacrylate, n-stearyl methacrylate, butoxydiethylene glycol methacrylate, methoxypolyethylene glycol methacrylate, cyclohexyl methacrylate, tetrahydrofurfuryl methacrylate, isobornyl methacrylate, 2-hydroxyethyl methacrylate, 2-hydroxypropyl methacrylate, 2-hydroxyethyl acrylate, 2-hydroxypropyl acrylate, 2-hydroxybutyl methacrylate, dimethylaminoethyl methacrylate, diethylaminoethyl methacrylate, 2-methacryloyloxyethylsuccinic acid, and glycidyl methacrylate. These may be used alone or in combination of two or more.

The (b) (meth)acrylic polymer having one or two (meth)acryloyloxy groups may be a (meth)acrylate polymerizable monomer not containing urethane structure. Specific examples of the (b) (meth)acrylic polymer having one or two (meth)acryloyloxy groups not containing urethane structure include 3,3,5-trimethylcyclohexyl acrylate, isobornyl acrylate, dicyclopentanyl acrylate, cyclohexyl methacrylate, tert-butyl methacrylate, 2-phenoxyethyl methacrylate, 3-phenoxybenzyl acrylate and triethylene glycol dimethacrylate.

As the (b) (meth)acrylic polymer having one or two (meth)acryloyloxy groups, specific examples of (meth)acrylic polymer containing one or more reactive groups selected from the group consisting of a hydroxyl group and a carboxylic acid group include hydroxy straight chain alkyl (meth)acrylate such as 2-hydroxyethyl (meth)acrylate, hydroxypropyl (meth)acrylate, mono-2-(methacryloyloxy) ethylsuccinic acid and 4-hydroxybutyl (meth)acrylate; hydroxyl group-containing (meth)acrylate such as caprolactone-modified hydroxy (meth)acrylate, hydroxy branched alkyl (meth)acrylate, mono(meth)acrylate of polyester diol obtained from dihydric carboxylic acid (such as phthalic acid) and dihydric alcohol (such as propylene glycol); monocarboxylic acid such as acrylic acid, methacrylic acid and crotonic acid; and tricarboxylic acid such as 4-[2-(methacryloyloxy)ethyl]-1,2,4-benzenetricarboxylate. These may be used alone or in combination of two or more.

The (b) (meth)acrylic polymer having one or two (meth)acryloyloxy groups may be used alone or in combination of two or more.

Specific examples of the (c) photoinitiator include an alkylphenone compound, an acetophenone compound, an acylphosphine oxide compound, a titanocene compound, an oxime ester compound, a benzoin compound, a benzophenone compound, a thioxanthone compounds, an α-acyloxime ester compound, phenylglyoxylate compound, a benzyl compound, an azo compound, a diphenyl sulfide compound, an organic dye compound, an iron-phthalocyanine compound, a benzoin ether compound, an anthraquinone compound, α-diketone, ketals, coumarins, α-aminoketone compound. Among these, alkylphenone compound, acetophenone compound, and acylphosphine oxide compound are preferable from the viewpoint of reactivity and the like. These may be used alone or in combination of two or more.

Specific examples of the alkylphenone compound used as the photopolymerization initiator include 2,2-dimethoxy-1,2-diphenylethan-1-one, 2-hydroxy-2-methyl-1-phenylpropane-1-one, 1-hydroxy cyclohexylphenylketone, 1-[4-(2-hydroxyethoxy)phenyl]-2-hydroxy-2-methyl-1-propan-1-one and the like.

Specific examples of acetophenone compounds used as the photopolymerization initiator include acetophenone, 2-hydroxy-2-phenylacetophenone, 2-ethoxy-2-phenylacetophenone, 2-methoxy-2-phenylacetophenone, 2-isopropoxy-2-phenylacetophenone, 2-i-butoxy-2-phenylacetophenone and 2-hydroxy-1-(4-(4-(2-hydroxy-2-methylpropionyl) benzyl) phenyl)-2-methylpropane-1 -one.

Specific examples of acylphosphine oxide compound used as photopolymerization initiators include 2,4,6-trimethylbenzoyl diphenylphosphine oxide, 2,6-dimethoxybenzoyl diphenylphosphine oxide, 2,6-dichlorobenzoyl diphenylphosphine oxide, 2,4,6-trimethylbenzoyl methoxyphenylphosphine oxide, 2,4,6-trimethylbenzoyl ethoxyphenylphosphine oxide, 2,3,5,6-tetramethylbenzoyl diphenylphosphine oxide and benzoyldi-(2,6)-dimethylphenyl) phosphonate. Examples of bisacylphosphine oxides include bis-(2,6-dichlorobenzoyl) phenylphosphine oxide, bis-(2,6-dichlorobenzoyl)-2,5-dimethylphenyl phosphine oxide, bis-(2,6-dichlorobenzoyl)-4-propylphenyl phosphine oxide, bis-(2,6-dichlorobenzoyl)-1-naphthylphosphine oxide, bis-(2,6-dimethoxybenzoyl) phenylphosphine oxide, bis-(2,6-dimethoxybenzoyl)-2,4,4-trimethylpentyl phosphine oxide, bis-(2,6-dimethoxybenzoyl)-2,5-dimethylphenyl phosphine oxide, bis-(2,4,6-trimethylbenzoyl) phenylphosphine oxide and (2,5,6-trimethylbenzoyl)-2,4,4-trimethylpentylphosphine oxide.

Further, the composition for stereolithography of the present invention may contain known additives. In the case that the purpose is adjustment of the color tone or paste properties, examples of these additives include pigments and dyes as colorants, organic solvents, thickeners, ultraviolet absorbers, polymerization inhibitors, fluorescent agents, inorganic fillers, and the additives in this case may be added in an amount of 0.0001 to 30 parts by weight based on the total weight of the composition. In particular, it is preferable that the composition contains 0.0001 to 2 parts by weight of one or more additives selected from colorants, ultraviolet absorber, polymerization inhibitors, and fluorescent agents, based on the total weight of the composition. In the present invention, it is possible that additives other than pigments, dyes, organic solvents, thickeners, ultraviolet absorbers, polymerization inhibitors, fluorescent agents, and inorganic fillers is not be contained.

When the purpose is to adjust the viscosity, it is possible to use a (meth)acrylic polymerizable compound, an acrylic resin, a polyester resin, an epoxy resin, a melamine resin, an olefin resin and the like that do not contain an allophanate group, as an additive. In the present invention, it is possibel that additives other than compounds having (meth)acrylic groups, acrylic resins, polyester resins, epoxy resins, melamine resins, and olefin resins are not contained.

Specific examples of such compound having (meth)acrylic group include 2-ethylhexyl (meth)acrylate, lauryl (meth)acrylate, stearyl (meth)acrylate, isobornyl (meth)acrylate, dipropylene glycol (meth)acrylate, ethoxydiethylene glycol (meth)acrylate, hydroxyethyl (meth)acrylate, 2-hydroxypropyl (meth)acrylate, tetrahydrofurfuryl (meth)acrylate, dicyclopentenyl (meth)acrylate, neopentyl glycol di(meth)acrylate, 1,6-hexanediol di(meth)acrylate, 1,9-nonanediol di(meth)acrylate, dimethylol tricyclodecane di(meth)acrylate, hydroxypivalic acid neopentyl glycol di(meth)acrylate, neopentyl glycol modified trimethylolpropane di(meth)acrylate, tripropylene glycol di(meth)acrylate, polypropylene glycol di(meth)acrylate, polyethylene glycol di(meth)acrylate, bis((meth)acryloxyethyl) bisphenol A, trimethylolpropane tri(meth)acrylate, ethylene oxide modified trimethylolpropane di(meth)acrylate, propylene oxide modified trimethylolpropane tri(meth)acrylate, glycerin tri(meth)acrylate, ethylene oxide modified glycerin tri(meth)acrylate, propylene oxide modified glycerin tri(meth)acrylate, ditrimethylolpropane tetra(meth)acrylate, pentaerythritol tri(meth)acrylate, pentaerythritol tetra(meth)acrylate, dipentaerythritol hexa(meth)acrylate, dipentaerythritol penta(meth)acrylate, polyfunctional (meth)acrylates in which a part of these (meth)acrylate compounds are substituted with alkyl groups or ε-caprolactone.

Furthermore, examples include polyester poly(meth)acrylate obtained by the reaction of polybasic acid such as phthalic acid and adipic acid, polyhydric alcohol such as ethylene glycol and butanediol, and (meth)acrylic acid compound; epoxy poly(meth)acrylate obtained by the reaction epoxy resin and (meth)acrylic acid compound; polysiloxane poly(meth)acrylate obtained by the reaction of polysiloxane and (meth)acrylic acid compound; and polyamide poly(meth)acrylate obtained by the reaction of polyamide and (meth)acrylic acid compound.

Furthermore, in the present invention, it is possible to contain various additives such as organic solvents, polymerization inhibitors, ultraviolet absorbers, light stabilizers, antioxidants, anti-yellowing agents, dyes, pigments, leveling agents, antifoaming agents, thickeners, antisettling agents, antistatic agents, anti-fogging agent, an anti-repellent agent, a wetting agent, a dispersing agent, an anti-sagging agent, and a coupling agent. In the present invention, it is possible that additives other than those described in this specification is not contained.

The dental photopolymerizable composition for 3D printer of the present invention can be used, for example, but not limited to, to prepare dental products, dental aligner materials, denture base materials, and dental mouthpieces.

### [Examples]

Hereinafter, the present invention will be described in detail with reference to Examples. However, the present disclosure is not limited to these Examples.

### [(a) (meth)acrylic polymerizable compound having an allophanate group]

### (a1) (meth)acrylic polymerizable compound having an allophanate group 1

A polyisocyanate resin was prepared from 1,6-hexamethylene diisocyanate and 1,3-butanediol. Specifically, it was prepared by the method disclosed in Comparative Example 1 of Japanese Unexamined Patent Application Publication No. H6-41270. The NCO content in the prepared polyisocyanate resin is 20.8 wt.%. Further, in the NMR analysis, almost no urethane groups were confirmed, and the amount of allophanate groups was 5 mol% and the amount of isocyanurate groups was 95 mol%. Then, 600 g of this polyisocyanate resin, 149 g of 2-hydroxyethyl methacrylate, 127 g of pentaerythritol triacrylate and 2 g of methylhydroquinone were charged into a reaction vessel and reacted at 80 °C for 4 hours under an air stream. By adding 0.6 g of dibutyltin laurate to this and further reacting at 80 °C for 4 hours to prepare (a1) (meth)acrylic polymerizable compound having an allophanate group 1. Further, the content of allophanate group determined by H'-NMR measurement using d6-DMSO as a solvent was 0.2 mmol/g. Furthermore, the viscosity measured using a rotational rheometer was 9000 mPa s. The glass transition point was 47 °C. It was confirmed that the obtained resin had three or more functional groups.

### (a2) (meth)acrylic polymerizable compound having an allophanate group 2

A polyisocyanate resin was prepared from 1,3-diisocyanato methylcyclohexane and isobutanol. Specifically, it was prepared by the method disclosed in Example 1 of Japanese Unexamined Patent Application Publication No. H6-41270. The NCO content in the prepared polyisocyanate resin is 18.2 wt.%. Further, in the NMR analysis, almost no urethane groups were confirmed, and the amount of allophanate groups was 35 mol% and the amount of isocyanurate groups was 75 mol%. Then, 600 g of this polyisocyanate resin, 241 g of 2-hydroxyethyl methacrylate, 206 g of pentaerythritol triacrylate and 2 g of methylhydroquinone were charged into a reaction vessel and reacted at 80 °C for 4 hours under an air stream. By adding 0.6 g of dibutyltin laurate to this and further reacting at 80 °C for 4 hours to prepare (a2) (meth)acrylic polymerizable compound having an allophanate group 2. Further, the content of allophanate group determined by H'-NMR measurement using d6-DMSO as a solvent was 1.0 mmol/g. Furthermore, the viscosity measured using a rotational rheometer was 60000 mPa s. The glass transition point was 98 °C. It was confirmed that the obtained resin had three or more functional groups.

### (a3) (meth)acrylic polymerizable compound having an allophanate group 3

A polyisocyanate resin was prepared from isophorone diisocyanate and isobutanol. Specifically, it was prepared by the method disclosed in Example 4 of Japanese Unexamined Patent Application Publication No. H6-41270. The NCO content in the prepared polyisocyanate resin is 11.6 wt.%. Further, in the NMR analysis, almost no urethane groups were confirmed, and the amount of allophanate groups was 60 mol% and the amount of isocyanurate groups was 40 mol%. Then, 600 g of this polyisocyanate resin, 149 g of 2-hydroxyethyl methacrylate, 127 g of pentaerythritol triacrylate and 2 g of methylhydroquinone were charged into a reaction vessel and reacted at 80 °C for 4 hours under an air stream. By adding 0.6 g of dibutyltin laurate to this and further reacting at 80 °C for 4 hours to prepare (a3) (meth)acrylic polymerizable compound having an allophanate group 3. Further, the content of allophanate group determined by H'-NMR measurement using d6-DMSO as a solvent was 0.5 mmol/g. Furthermore, the viscosity measured using a rotational rheometer was 32000 mPa s. The glass transition point was 120 °C. It was confirmed that the obtained resin had three or more functional groups.

### (a4) (meth)acrylic polymerizable compound having an allophanate group 4

Hexane, 1,6-diisocyanate, homopolymer, and 2-hydroxyethyl acrylate block were used. The viscosity measured using a rotational rheometer was 60000 mPa s. It has a glass transition point of 98°C and is an acrylate resin containing a tetrafunctional allophanate group.

### (a5) (meth)acrylic polymerizable compound having an allophanate group 5

Hexane, 1,6-diisocyanate, homopolymer, 2-hydroxyethyl acrylate and propylene glycol monoacrylate block were used. The viscosity measured using a rotary rheometer is 30000 mPa·s. It has a glass transition point of 118 °C, and is an acrylate resin containing trifunctional allophanate groups.

### [(b) (meth)acrylic polymer having one or two (meth)acryloyloxy groups]

### <(meth)acrylic polymer not containing urethane structure and having one (meth)acryloyloxy group>

### (b1) mono-2-(methacryloyloxy)ethylsuccinic acid

### (b2) 3-phenoxybenzyl acrylate

<(meth)acrylic polymer not containing urethane structure and having two (meth)acryloyloxy groups>

### (b3) triethylene glycol dimethacrylate

### <(meth)acrylic polymer having urethane structure and having two (meth)acryloyloxy groups>

### (b4) di (methacryloxyethyl) trimethylhexamethylene diurethane

### <(meth)acrylic polymer having three (meth)acryloyloxy groups>

### trimethylolpropane trimethacrylate

### [(c) photoinitiator]

### <acylphosphine oxide compound>

### (c1) monoacylphosphine oxide

### <acetophenone compound>

### (c2) 2-hydroxy-1-(4-(4-(2-hydroxy-2-methylpropionyl) benzyl) phenyl)-2-methylpropan-1-one

### [(d) (meth)acrylic polymerizable compound not containing an allophanate group]

### (d1) di(methacryloxyethyl) trimethyl hexamethylene diurethane

### [Preparation of composition for stereolithography]

As described in the tables of Examples, the components including the (a) (meth)acrylic polymerizable compound having an allophanate group, the (b) (meth)acrylic polymer having one or two (meth)acryloyloxy groups, and the (c) photoinitiator were added in a predetermined amount into a container and mixed using a rotation/revolution mixer ARV-310 (manufactured by Syncy Corporation) to prepare a photopolymerizable composition.

### [Viscosity measurement method]

Viscosity of the photopolymerizable composition was measured by using a rotary rheometer (Physica MCR301, manufactured by Anton Paar) under the conditions of temperature: 23 °C and rotation speed: 20 rpm.

### [Glass transition point measurement method]

By using DMA Q800 (manufactured by TA Instruments), measurements were performed in tensile mode on a sample length of 20 mm. The composition for stereolithography was poured into a mold (width 10 mm x thickness 1 mm x length 50 mm), and polymerized and cured using a dental light irradiator (SOLIDILIGHT LED, manufactured by SHOFU INC.) to prepare a sample. The glass transition point (Tg) of the sample was measured from the peak top temperature of the loss tangent (tanδ).

### [Formability]

The photopolymerizable composition prepared in the above [Preparation of composition for stereolithography] was modeled into the shapes of various test specimens using a dental 3D printer DWP-80S (manufactured by DG SHAPE). After modeling, the modeled object was washed with ethanol for 10 minutes and dried. After storage for 24 hours, post-polymerization was performed for 10 minutes by a dental light irradiator (SOLIDILIGHT LED, manufactured by SHOFU INC.). The surface of the test object was visually checked. Evaluation criteria were as follows.
A: There were no modeling defects and it was good.
B: There was slight surface roughness and it was modelable.
C: The surface of the modeled object was extremely rough or it was difficult to prepare the modeled object

### [Impact resistance]

The composition for stereolithography was poured into a mold (width 4 mm x thickness 6 mm x length 50 mm), polymerized and cured using a dental light irradiator (SOLIDILIGHT LED, manufactured by SHOFU INC.), and machied by using a milling machine to form V-shaped notch with a depth of 1.2 mm, and used as a test specimen. By using an impact tester "IMPACT TESTER" (manufactured by Toyo Seiki Seisaku-sho), the load at which the test specimen broke was measured using a hammer capacity of 0.5 J. The measured values were converted into units of J/m², and the average value was calculated as impact resistance. The measured value of 0.8 J/m² or more were judged to have excellent impact resistance.

### [Color tone]

A plate-shaped test specimen with a diameter of 15 mm and a thickness of 1.0 mm was prepared according to the test method described in the above [Formability]. After preparation, it was stored at room temperature for 14 days and used as a test specimen. The L*a*b values in the white background were measured using a spectrophotometer (CM-3500d: Konica Minolta). Those having b* value of 4 or less were considered good because they have very little yellowing and are extremely transparent. Those having b* value of 10 or less were considered usable because they have little yellowing and are excellent in transparency. Those having b* value exceeding 10 were considered non-conforming.

**[Table 1]**

| | | | Example 1 | Example 2 | Example 3 | Example 4 | Example 5 | Example 6 | Example 7 | Example 8 | Example 9 | Example 10 |
|---|---|---|---|---|---|---|---|---|---|---|---|---|
| | (a) (meth)acrylic polymerizable compound having an allophanate group | (a1) | 70 | 70 | | | 41 | 79 | 35 | 90 | 31 | 98 |
| | | (a2) | | | | | | | | | | |
| | | (a3) | | | | | | | | | | |
| | | (a4) | | | 70 | | | | | | | |
| | | (a5) | | | | 70 | | | | | | |
| | (b) (meth)acrylic polymer having one or two (meth)acryloyloxy groups | (b1) mono-2-(methacryloyloxy) ethylsuccinic add | 25 | 1 | 15 | 15 | 24 | 20 | 25 | 1 | 24 | 2 |
| | | (b2) 3-phenoxybenzyl acrylate | | | | | | | | | | |
| | | (b3) triethylene glycol dimethacrylate | 5 | 29 | 15 | 15 | 35 | 1 | 40 | 9 | 45 | |
| Component | | (b4) di (methacryloxyethyl) trimethylhexamethylene diurethane | | | | | | | | | | |
| | (meth)acrylic polymer having three (meth)acryloyloxy groups | trimethylolpropane trimethacrylate | | | | | | | | | | |
| | (c) photoinitiator | (c1) monoacylphosphine oxide | 2 | 3 | 3 | 3 | 2 | 2 | 2 | 2 | 2 | 3 |
| | | (c2) 2-hydrory-1-(4-(4-(2-hydroxy-2-methylpropionyl) benzyl) phenyl)-2-methylpropan-1-one | | | | | | | | | | |
| | (d) (meth)acryilc polymerizable compound not containing an allophanate group | (d1) d1(methacryloxyethyl) trimethyl hexamethylene dlurethane | | | | | | | | | | |
| Result | Viscosity of composition | | 1900 | 2200 | 3700 | 2400 | 150 | 2300 | 140 | 4300 | 70 | 7800 |
| | Glass transition point of cured composition | | 37 | 45 | 70 | 98 | 64 | 32 | 72 | 62 | 73 | 45 |
| | Formability | | A | A | A | A | A | A | A | A | A | A |
| | Impact resistance | | 3.1 | 1.6 | 1.4 | 1.2 | 1.5 | 1.5 | 1.4 | 1.4 | 1.3 | 1.4 |
| | Color tone | | 3.9 | 3.8 | 3.9 | 4 | 3.9 | 3.7 | 4.1 | 4.1 | 5.2 | 4.4 |

**[Table 2]**

| | | | Example 11 | Example 12 | Example 13 | Example 14 | Example 15 | Example 16 | Example 17 | Example 18 | Example 19 | Example 20 |
|---|---|---|---|---|---|---|---|---|---|---|---|---|
| | (a) (meth)acrylic polymerizable compound having an allophanate group | (a1) | 98 | 70 | 70 | 70 | 70 | 70 | 70 | 29 | 70 | 70 |
| | | (a2) | | | | | | | | | | |
| | | (a3) | | | | | | | | | | |
| | | (a4) | | | | | | | | | | |
| | | (a5) | | | | | | | | | | |
| | (b) (meth)acrylic polymer having one or two (meth)acryloyloxy groups | (b1) mono-2-(methacryloyloxy) ethylsuccinic acid | | 1 | 1 | 1 | 1 | 1 | 1 | 1 | 25 | 25 |
| | | (b2) 3-phenoxybenzyl acrylate | | | | | | | | | | |
| | | (b3) triethylene glycol dimethacrylate | 2 | 29 | 29 | 29 | 29 | 29 | 29 | 70 | 5 | 5 |
| Component | | (b4) di (methacryloxyethyl) trimethylhexamethylene diurethane | | | | | | | | | | |
| | (meth)acrylic polymer having three (meth)acryloyloxy groups | trimethylolpropane trimethacrylate | | | | | | | | | | |
| | (c) photoinitiator | (c1) monoacylphosphine oxide | 3 | 1 | 4 | 0.5 | 5 | 1.5 | 0.01 | 3 | 0.009 | 5.1 |
| | | (c2) 2-hydrory-1-(4-(4-(2-hydroxy-2-methylpropionyl) benzyl)phenyl)-2-methylpropan-1-one | | | | | | 1.5 | | | | |
| | (d) (meth)acrylic polymerizable compound not containing an allophanate group | (d1) di(methacryloxyethyl) trimethyl hexamethylene diurethane | | | | | | | | | | |
| Result | Viscosity of composition | | 7900 | 2200 | 2200 | 2100 | 2300 | 2200 | 2200 | 100 | 1800 | 1800 |
| | Class transition point of cured composition | | 50 | 71 | 72 | 70 | 73 | 68 | 65 | 100 | 66 | 67 |
| | Formability | | A | A | A | A | A | A | B | A | B | A |
| | Impact resistance | | 1.4 | 1.5 | 1.6 | 1.4 | 1.4 | 1.5 | 1.3 | 0.9 | 3.2 | 3.1 |
| | Color tone | | 4.6 | 3.8 | 3.9 | 1.8 | 4.5 | 3.8 | 1.5 | 6.0 | 1.8 | 6.5 |

**[Table 3]**

| | | | Example 21 | Example 22 | Example 23 | Example 24 | Example 25 | Example 26 | Example 27 | Comparative Example 1 | Comparative Example 2 |
|---|---|---|---|---|---|---|---|---|---|---|---|
| | (a) (meth)acryllc polymerizable compound having an allophanate group | (a1) | | 50 | 50 | | 70 | 70 | | | 70 |
| | | (a2) | | | | 65 | | | | | |
| | | (a3) | 50 | | | | | | | | |
| | | (a4) | | | | | | | 70 | | |
| | | (a5) | | | | | | | | | |
| | (b) (meth)acryllc polymer having one or two (meth)acryloyloxy groups | (b1) mono-2-(methacryloyloxy) ethylsuccinic add | 25 | | 50 | 25 | | | | 1 | |
| | | (b2) 3-phenoxybenzyl acrylate | | | | | 25 | 30 | 15 | | |
| | | (b3) trlethylene glycol dimethacrylate | 25 | | | 10 | 5 | | 15 | 29 | |
| Component | | (b4) di (methacryloxyethyl) trimethylhexamethylene diurethane | | 50 | | | | | | | |
| | (meth)acryllc polymer having three (meth)acryloyloxy groups | trlmethylolpropane trimethacrylate | | | | | | | | | 30 |
| | (c) photoinitiator | (c1) monoacylphosphlne oxide | 3 | 3 | 3 | 2 | 2 | 2 | 3 | 3 | 3 |
| | | (c2) 2-hydrory-1-(4-(4-(2-hydroxy-2-methylproplonyl) benzyl) phenyl)-2-methylpropan-1-one | | | | | | | | | |
| | (d) (meth)acryllc polymerizable compound not containing an allophanate group | (d1) di(methacryloryethyl) trimethyl hexamethylene diure thane | | | | | | | | 70 | |
| Result | Viscosity of composition | | 2300 | 7900 | 1400 | 28000 | 1800 | 1800 | 3600 | 400 | 6800 |
| | Glass transition point of cured composition | | 63 | 53 | 59 | 83 | 36 | 35 | 69 | 143 | 120 |
| | Formability | | **A** | B | B | B | A | A | A | **A** | **C** |
| | Impact resistance | | 0.9 | 0.9 | 4.0 | 0.8 | 3.1 | 3.3 | 1.5 | 0.6 | 0.7 |
| | Color tone | | 6.6 | 4.1 | 3.8 | 6.7 | 3.7 | 3.6 | 3.9 | 13 | 15 |

### [Details of various compositions]

In Examples 1 to 17 and 25 to 27 in the tables, high impact resistance and good toughness were confirmed. Moreover, the color tone (b* value) also showed a value of 5.5 or less, and it was recognized that the transparency is excellent.

In Example 18 in the table, since the (a) (meth)acrylic polymerizable compound having an allophanate group was small, impact resistance was low and toughness slightly decreased. Furthe, although it exhibited a high glass transition point, it was found that it exhibited good physical properties as a whole.

In Example 19 in the table, since the amount of (c) photoinitiator was small, it was confirmed that it was difficult to cure and the formability was slightly decreased, but it exhibited good physical properties as a whole.

In Example 20 in the table, since the amount of the (c) photoinitiator is large, a tendency that the color tone (b* value) became high were shown, but it was observed that it exhibited good physical properties as a whole.

In Example 19 in the table, the (a) (meth)acrylic polymerizable compound having an allophanate group has a glass transition point of 120°C. Although a tendency that the impact resistance was low, the toughness was slightly decreased, and the color tone (b* value) was high were shown, but it was observed that it exhibited good physical properties as a whole.

In Example 22 in the table, the (b) (meth)acrylic polymer having one or two (meth)acryloyloxy groups contained urethane structure. Although the viscosity of the composition for stereolithography increased and the formability decreased slightly, it was observed that it exhibited good physical properties as a whole.

In Example 23 in the table, the (b) (meth)acrylic polymer having one or two (meth)acryloyloxy groups consisted of only (meth)acrylic polymer having one (meth)acryloyloxy group and was contained in large amounts. Although the modeled object became soft and the formability decreased slightly, it was observed that it exhibited good physical properties as a whole.

In Example 24 in the table, the viscosity of the composition for stereolithography was high. Although the formability decreased, it was observed that it exhibited good physical properties as a whole.

In Comparative Example 1, the (a) (meth)acrylic polymerizable compound having an allophanate group was not contained. Impact resistance was low and toughness could not be imparted. Furthermore, the color tone (b* value) was high, the transparency was poor, and good physical properties could not be obtained as a whole.

In Comparative Example 2, the (b) (meth)acrylic polymer having one or two (meth)acryloyloxy groups was not contained, and only (meth)acrylic polymer having a polyfunctional (meth)acryloyloxy group was contained. Impact resistance was low and toughness could not be imparted. Furthermore, the color tone (b* value) was high, the transparency was poor, and good physical properties could not be obtained as a whole.

In addition, it was confirmed that, similar results were obtained even when the components further contained 0.1 part by weight of the coloring material and the ultraviolet absorber in the examples of the present description.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this invention without difficulty. Accordingly, any such changes and modifications are intended to be included in the scope of the embodiments.

### [Industrial applicability]

The present invention can be used in the industry to prepare a dental restoration with 3D printer using photopolymerizable compositions.

## Claims

1. A dental photopolymerizable composition for 3D printer, comprising
(a) (meth)acrylic polymerizable compound having an allophanate group,
(b) (meth)acrylic polymer having one or two (meth)acryloyloxy groups, and
(c) photoinitiator.

2. The dental photopolymerizable composition for 3D printer according to claim 1, wherein the dental photopolymerizable composition for 3D printer comprises
30 to 99% by weight of the (a) (meth)acrylic polymerizable compound having an allophanate group,
1 to 70% by weight of the (b) (meth)acrylic polymer having one or two (meth)acryloyloxy groups, and
a content of the (c) photoinitiator is 0.01 to 5 parts by weight with respect to 100 parts by weight of the total of the (a) (meth)acrylic polymerizable compound having an allophanate group and the (b) (meth)acrylic polymer having one or two (meth)acryloyloxy groups.

3. The dental photopolymerizable composition for 3D printer according to claim 1 or 2, wherein
the (a) (meth)acrylic polymerizable compound having an allophanate group has a viscosity of 60,000 mPa s or less measured by a rotary rheometer under the conditions of temperature: 23 °C and rotation speed: 20 rpm.

4. The dental photopolymerizable composition for 3D printer according to any one of claims 1 to 3, wherein
a glass transition temperature of a cured product of the (a) (meth)acrylic polymerizable compound having an allophanate group is 120 °C or less.

5. The dental photopolymerizable composition for 3D printer according to any one of claims 1 to 4, wherein
the (a) (meth)acrylic polymerizable compound having an allophanate group has three or more (meth)acryloyloxy groups.

6. The dental photopolymerizable composition for 3D printer according any one of claims 1 to 5, wherein
the (b) (meth)acrylic polymer having one or two (meth)acryloyloxy groups does not contain a urethane structure.

7. The dental photopolymerizable composition for 3D printer according to any one of claims 1 to 6, wherein
the (b) (meth)acrylic polymer having one or two (meth)acryloyloxy groups contains at least one (meth)acrylic polymer having two (meth)acryloyloxy groups.

8. The dental photopolymerizable composition for 3D printer according to any one of claims 1 to 7, wherein
the dental photopolymerizable composition for 3D printer comprises 1 to 25 parts by weight of a (meth)acrylic polymer having one (meth)acryloyloxy group with respect to 100 parts by weight of the total of the (a) (meth)acrylic polymerizable compound having an allophanate group and the (b) (meth)acrylic polymer having one or two (meth)acryloyloxy groups.

9. The dental photopolymerizable composition for 3D printer according to any one of claims 1 to 8, wherein
the (b) (meth)acrylic polymer having one or two (meth)acryloyloxy groups contains a (meth)acrylic polymer containing one or more reactive groups selected from the group consisting of a hydroxyl group and a carboxylic acid group.

10. The dental photopolymerizable composition for 3D printer according to any one of claims 1 to 9, wherein
the (c) the photoinitiator is one or more selected from the group consisting of an alkylphenone compound, an acetophenone compound and an acylphosphine oxide compound.

11. The dental photopolymerizable composition for 3D printer according to any one of claims 1 to 10, wherein
the dental photopolymerizable composition for 3D printer has a viscosity of 10,000 mPa s or less measured by a rotary rheometer under the conditions of temperature: 23 °C and rotation speed: 20 rpm.

12. The dental photopolymerizable composition for 3D printer according to any one of claims 1 to 11, wherein
a glass transition temperature of a cured product of the dental photopolymerizable composition for 3D printer is within a range of 30 to 100 °C.

13. The dental photopolymerizable composition for 3D printer according to any one of claims 1 to 12, wherein
the dental photopolymerizable composition for 3D printer further comprises an additive.

14. The dental photopolymerizable composition for 3D printer according to claim 13, wherein
the additive includes one or more selected from the group consisting of a colouring agent, an ultraviolet absorber, a polymerization inhibitor and a fluorescent agent.

15. The dental photopolymerizable composition for 3D printer according to claim 13, wherein
the dental photopolymerizable composition for 3D printer comprises 0.0001 to 2 parts by weight of the additive with respect to 100 parts by weight of the dental photopolymerisable composition for 3D printers excluding the additive.

16. A dental product prepared by a stereolithographic 3D printer using a dental photopolymerisable composition for 3D printer according to any one of claims 1 to 15.

17. A dental aligner material prepared by a stereolithographic 3D printer using a dental photopolymerisable composition for 3D printer according to any one of claims 1 to 15.

18. A denture base material prepared by a stereolithographic 3D printer using a dental photopolymerisable composition for 3D printer according to any one of claims 1 to 15.

19. A dental mouthpiece prepared by a stereolithographic 3D printer using a dental photopolymerisable composition for 3D printer according to any one of claims 1 to 15.
